(19) Europäisches Patentamt
European Patent Office
Office européen des brevets

(11) Veröffentlichungsnummer: **0 123 123**
**A2**

# (12) EUROPÄISCHE PATENTANMELDUNG

(21) Anmeldenummer: **84102968.9**

(22) Anmeldetag: **17.03.84**

(51) Int. Cl.³: **C 07 D 263/12**
C 07 D 263/14

(30) Priorität: **25.03.83 DE 3310905**

(43) Veröffentlichungstag der Anmeldung:
**31.10.84 Patentblatt 84/44**

(84) Benannte Vertragsstaaten:
**AT BE CH DE FR GB IT LI LU NL SE**

(71) Anmelder: **Henkel Kommanditgesellschaft auf Aktien**
**Postfach 1100 Henkelstrasse 67**
**D-4000 Düsseldorf-Holthausen(DE)**

(72) Erfinder: **Schlüter, Kaspar, Dr.**
**Spreestrasse 26**
**D-5650 Solingen 11(DE)**

(72) Erfinder: **Schieferstein, Ludwig, Dr.**
**Posener Strasse 70**
**D-5600 Wuppertal 2(DE)**

(54) **Verfahren zur Herstellung von 2-Isopropenyloxazolinen.**

(57) Die Erfindung betrifft ein Verfahren zur Herstellung von 2-Isopropenyloxazolinen der allgemeinen Formel (I)

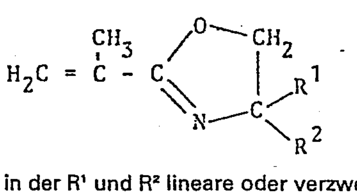

(I)

in der R¹ und R² lineare oder verzweigte, gegebenenfalls mit Hydroxy- und/oder Alkoxygruppen substituierte Alkylreste mit 1–8 C-Atomen oder Phenylreste sein können, die gegebenenfalls mit Hydroxy- und/oder Alkoxygruppen substituiert sein können, das dadurch gekennzeichnet ist, daß man Methacrylnitril mit 2-Aminoalkoholen der allgemeinen Formel (II)

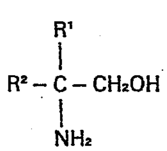

(II)

in der R¹ und R² die oben angegebene Bedeutung haben können, bei erhöhter Temperatur in Gegenwart eines Katalysators umsetzt.

EP 0 123 123 A2

Croydon Printing Company Ltd.

Henkelstraße 67
4000 Düsseldorf, den 13. Dez. 1983

0123123
HENKEL KGaA
ZR-FE/Patente
AvK/Dr.SchOe

Patentanmeldung D 6710 EP
Verfahren zur Herstellung von
2-Isopropenyloxazolinen

---

Gegenstand der Erfindung ist ein Verfahren zur Herstellung von 2-Isopropenyloxazolinen der allgemeinen Formel (I)

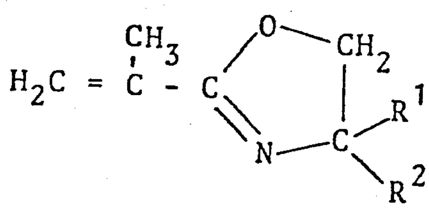

(I)

in der $R^1$ und $R^2$ lineare oder verzweigte, gegebenenfalls mit Hydroxy-, und/oder Alkoxygruppen substituierte Alkylreste mit 1 - 8 C-Atomen oder Phenylreste sein können, die gegebenenfalls mit Hydroxy- und/oder Alkoxygruppen substituiert sein können,das dadurch gekennzeichnet ist, daß man Methacrylnitril mit 2-Aminoalkoholen der allgemeinen Formel (II)

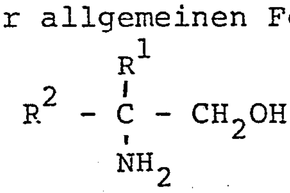

(II)

in der $R^1$ und $R^2$ die oben angegebene Bedeutung haben können, bei erhöhter Temperatur in Gegenwart eines Katalysators umsetzt.

2-Isopropenyloxazoline sind in der Polymertechnik nützliche Verbindungen aufgrund ihrer hohen Reaktivität in radikalischen Polymerisationsreaktionen. Sie eignen sich besonders als Monomere zum Einsatz in radikalisch härtenden Reaktivklebstoffen.

Im Rahmen der bisher bekannten Verfahren zur Herstellung von 2-Isopropenyloxazolinen wurden oft teure oder nur schwer zugängliche Ausgangschemikalien verwendet. Mehrstufige, zum Teil aufwendige Verfahren erbrachten in allen Fällen niedrige Gesamtausbeuten im Verhältnis zu den eingesetzten Reagenzien. Zudem war der Reinigungsprozeß der Produkte besonders dann sehr aufwendig, wenn Nebenreaktionen zu Produktmischungen mit hohem Anteil an Nebenprodukt führten.

So wird in der US-PS 3 505 297 die Umsetzung von Methacrylnitril mit 2-Chlorethanol in einer Pinner-Reaktion zum Imidoester beschrieben, der in Gegenwart einer starken Base zu 2-Isopropenyloxazolin cyclisiert. Die Produktausbeuten liegen, je nach Substitution in 4- bzw. 5-Stellung des Rings, zwischen 33 und 43 %.

Verfahren zur Herstellung von 2-Isopropenyloxazolin (I, $R^1$=$R^2$=H) und seinen Derivaten aus Oxazolinen, die in 2-Stellung durch eine Alkylgruppe bzw. substituierte Alkylgruppe substituiert sind, beschreiben die DE-OSen 23 02 168 und 27 27 824. Die Ausgangsstoffe dieser Verfahren müssen jedoch als relativ teuer und im technischen Maßstab nicht ohne weiteres zugänglich bezeichnet werden, so daß die Herstellung von 2-Isopropenyloxazolinen für großtechnische Verfahren aus ökonomischen Gründen nicht infrage kommt. Aufgrund möglicher Nebenreaktionen sind auch die Ausbeuten unbefriedigend.

P.L. De Benneville, L.S. Luskin und H.J. Sims (Journ. Org.Chem. 23, 1355 (1958) erhielten 2-Isopropenyl-4,4-dimethyloxazolin (I, $R^1$=$R^2$=$CH_3$) durch Umsetzung von Methacrylsäuremethylester mit 2-Amino-2-methylpropanol. Zwar betrug die Produktausbeute dieses Verfahrens 60%,

aber zur Isolierung des reinen Oxazolins war ein mehrstufiger, aufwendiger Reinigungsprozeß notwendig.

Aufgrund der breiten Verwendbarkeit des 2-Isopropenyloxazolins und seiner Derivate als Monomere für radikalische Polymerisationsreaktionen, besonders für radikalisch härtende Reaktivklebstoffe, besteht also ein Bedarf nach einer einfachen, wirtschaftlichen, den bekannten Verfahren überlegenen Synthese für 2-Isopropenyloxazoline.

Es wurde nun überraschend gefunden, daß man 4,4-disubstituierte 2-Isopropenyloxazoline der allgemeinen Formel (I) in einem einstufigen Verfahren durch Umsetzung von Methacrylnitril mit 2-Aminoalkoholen der allgemeinen Formel (II) bei erhöhten Temperaturen in Gegenwart eines Katalysators in hohen Ausbeuten und guter Reinheit erhalten kann.

Demgemäß betrifft die Erfindung ein Verfahren zur Herstellung von 2-Isopropenyloxazolinen der allgemeinen Formel (I)

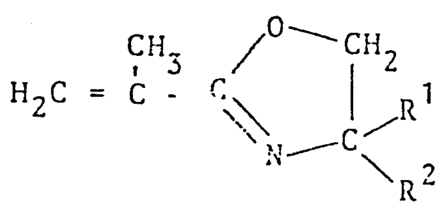 (I)

in der $R^1$ und $R^2$ lineare oder verzweigte, gegebenenfalls mit Hydroxy- und/oder Alkoxygruppen substituierte Alkylreste mit 1 - 8 C-Atomen oder Phenylreste sein können, die gegebenenfalls mit Hydroxy- und/oder Alkoxygruppen substituiert sein können, das dadurch gekennzeichnet ist, daß man Methacrylnitril mit 2-Aminoalkoholen der allgemeinen Formel (II)

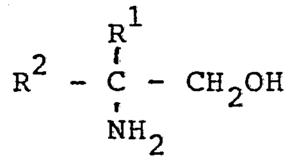

(II)

in der $R^1$ und $R^2$ die oben angegebene Bedeutung haben können, bei erhöhter Temperatur in Gegenwart eines Katalysators umsetzt.

Entsprechend dem erfindungsgemäßen Verfahren werden Methacrylnitril und 2-Aminoalkohole der allgemeinen Formel (II) in einem molaren Verhältnis von 1,5 bis 1,05 : 1, bevorzugt 1,25 bis 1,05 : 1, bei erhöhten Temperaturen in Abwesenheit eines Lösungsmittels miteinander zur Umsetzung gebracht. Das dabei einzuhaltende Temperaturniveau richtet sich nach den Siedepunkten der beteiligten Reaktionskomponenten, liegt jedoch in allen Fällen im Bereich zwischen 100 und 150°C.

Das Verfahren gemäß der vorliegenden Erfindung, in dem gemäß nachfolgendem Reaktionsschema Methacrylnitril mit 2-Aminoalkoholen in einem Reaktionsschritt zu 2-Isopropenyloxazolinen reagiert, wird in Gegenwart eines Katalysators durchgeführt.

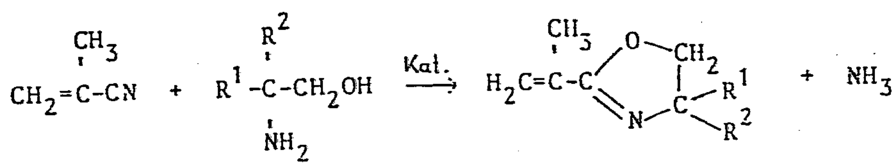


II I

Als Katalysatoren werden z.B. Protonensäuren wie $H_2SO_4$ oder Salze, wie Kupfersulfat, Zinkchlorid oder Lithiumchlorid verwendet, aber auch Salze organischer Säuren wie Cadmiumacetat, Zinkacetat oder Kupferacetat. Von diesen werden die Zink- und Cadmiumsalze bevorzugt eingesetzt. Die erfindugnsgemäß der Reaktionsmischung zuzusetzenden Mengen liegen im Bereich von 0,1 - 8 Mol-%, bevorzugt 2 - 3 Mol-%, bezogen auf Methacrylnitril.

Um die unkontrollierte Polymerisation der gebildeten 2-Isopropenyloxazoline unter den Bedingungen der Reaktion zu verhindern, wird den Reaktionsmischungen eine geringe Menge eines nicht flüchtigen Polymerisationsinhibitors, z.B. Bis(2-hydroxy-4-methyl-6-cyclohexylphenyl)methan zugsetzt.

Ein besonderer Vorteil des erfindungsgemässen Verfahrens ist es, daß die gewünschten 2-Isopropenyloxazoline in einem Reaktionsschritt gewonnen werden können, ohne daß die Bildung größerer Mengen an Nebenprodukten beobachtet wird. Unter den gegebenen Bedingungen verläuft die Reaktion mit Ausbeuten zwischen 70 und 90 %, bezogen auf den im Unterschuß eingesetzten 2-Aminoalkohol. Durch Destillation bei Normaldruck oder geringem Unterdruck lassen sich die Produkte in hoher Reinheit isolieren.

Das erfindungsgemäße Verfahren wird durch die nachstehenden Beispiele näher erläutert.

Beispiel 1

2-Isopropenyl-4,4-dimethyloxazolin

In einem 500 ml-Dreihalskolben mit Tropftrichter, Rührer und Rückflußkühler wurden 107,3 g (1,6 mol) Methacrylnitril, 10,7 g Cd $(CH_3CO_2)_2 \cdot 2H_2O$ (0,04 mol) und 2,5 g

eines nicht-flüchtigen Polymerisationsinhibitors (Bis-(2-hydroxy-4-methyl-6-cyclohexylphenyl)methan) und 44,5 g (0,5 mol) 2-Amino-2-methylpropanol vorgelegt und auf 100°C erhitzt. Nach Einsetzen der $NH_3$-Gasentwicklung wurden weitere 89 g (1,0 mol) 2-Amino-2-methylpropanol im Verlauf von 1,5 h zugetropft. Anschließend wurde noch 22 h am Rückfluß gekocht - die Temperatur stieg dabei bis auf ca 130°C - und 2-Isopropenyl-4,4-dimethyloxazolin (I, $R^1=R^2=CH_3$) durch fraktionierte Destillation bei Normaldruck isoliert. Bei 149 - 152°C erhielt man 150,1 g (1,08 mol, 72 % der theoretischen Ausbeute) 2-Isopropenyl-4,4-dimethyloxazolin in einer gaschromatographischen Reinheit von 92 %.

Beispiel 2

In einem 1-l-Dreihalskolben mit Tropftrichter, Rührer und Rückflußkühler wurden 238,4 g (2,0 mol) 2-Amino-2-ethyl-propandiol-1,3, 13,3 g (0,05 mol) $Cd(CH_3CO_2)_2 \cdot 2H_2O$ und 4,1 g Polymerisationsinhibitor (Bis-(2-hydroxy-4-methyl-6-cyclohexylphenyl)methan) vorgelegt und auf 130°C erhitzt. Bei dieser Temperatur wurden 167,5 g (2,5 mol) Methacrylnitril innerhalb von 0,5 h zugetropft. Dabei fiel die Temperatur auf 92°C ab und eine schwache $NH_3$-Gasentwicklung setzte ein. Man ließ noch 30 h nachreagieren, wobei nach ca 20 h die Temperatur wieder auf 130°C gestiegen war und für die restlichen 10 h bei diesem Wert belassen wurde. Das Produkt wurde aus dem rohen Reaktionsgemisch durch fraktionierte Destillation isoliert. Man erhielt bei 83 - 86°C/0,3 Torr 276,2 g (1,63 mol, 82 % d.theor.Ausbeute) 2-Isopropenyl-4-ethyl-4-hydroxymethyloxazolin in einer gaschromatographischen Reinheit von 92 %.

Beispiel 3

Beispiel 1 wurde unter sonst gleichen Bedingungen mit verschiedenen Katalysatoren und Katalysatormengen wiederholt.

| Katalysator | Menge bezogen auf Methacrylnitril | Ausbeute |
| --- | --- | --- |
| $Cd(CH_3CO_2)_2.2H_2O$ | 0,1 Mol-% | 12 % |
| | 1,0 Mol-% | 60 % |
| | 5,0 Mol-% | 71 % |
| $Zn(CH_3CO_2)_2.2H_2O$ | 2,5 Mol-% | 67 % |
| $ZnCl_2$ | 2,5 Mol-% | 44 % |
| LiCl | 2,5 Mol-% | 53 % |
| $CuSO_4$ | 2,5 Mol-% | 49 % |

## Patentansprüche

1. Verfahren zur Herstellung von 2-Isopropenyloxazolinen der allgemeinen Formel (I)

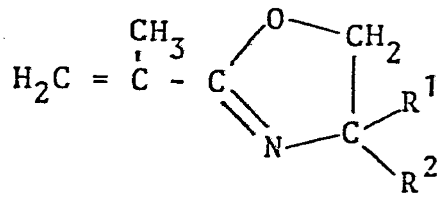

(I)

in der $R^1$ und $R^2$ lineare oder verzweigte, gegebenenfalls mit Hydroxy- und/oder Alkoxygruppen substituierte Alkylreste mit 1 - 8 C-Atomen oder Phenylreste sein können, die gegebenenfalls mit Hydroxy- und/oder Alkoxygruppen substituiert sein können, dadurch gekennzeichnet, daß man Methacrylnitril mit 2-Aminoalkoholen der allgemeinen Formel (II)

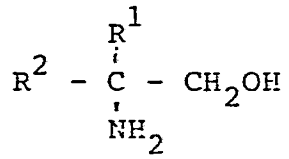

(II)

in der $R^1$ und $R^2$ die oben angegebene Bedeutung haben können, bei erhöhter Temperatur in Gegenwart eines Katalysators umsetzt.

2. Verfahren zur Herstellung von 2-Isopropenyloxazolinen nach Anspruch 1, dadurch gekennzeichnet, daß die Reaktion bei Temperaturen von 90 bis 150°C, bevorzugt bei 130°C, durchgeführt wird.

3. Verfahren nach Ansprüchen 1 und 2, dadurch gekennzeichnet, daß als Katalysatoren Schwefelsäure, Kupfersulfat, Zinkchlorid, Lithiumchlorid, Kupferacetat, Zinkacetat oder Cadmiumacetat, bevorzugt Zinkacetat oder Cadmiumacetat verwendet werden.

4. Verfahren nach Ansprüchen 1 - 3, dadurch gekennzeichnet, daß die Reaktion in Abwesenheit eines Lösungsmittels durchgeführt wird.

5. Verfahren nach Ansprüchen 1 - 4, dadurch gekennzeichnet, daß der Reaktionsmischung eine geringe Menge eines nicht flüchtigen Polymerisationsinhibitors zugegeben wird.

6. Verfahren nach Ansprüchen 1 - 5, dadurch gekennzeichnet, daß die Katalysatormenge 0,1 bis 8 Mol-%, bezogen auf Methacrylnitril, bevorzugt 1 bis 3 Mol-%, beträgt.